# EUROPÄISCHE PATENTSCHRIFT

(11) **EP 0 651 762 B1**
(45) Veröffentlichungstag und Bekanntmachung des Hinweises auf die Patenterteilung: **17.11.1999**
(21) Anmeldenummer: 91914577.1
(22) Anmeldetag: 12.08.1991
(51) Int. Cl.: C07K 1/04, C07H 21/00, B01J 19/00

(54) **VERFAHREN ZUR SCHNELLEN SYNTHESE VON TRÄGERGEBUNDENEN ODER FREIEN PEPTIDEN ODER OLIGONUCLEOTIDEN, DAMIT HERGESTELLTES FLACHMATERIAL, DESSEN VERWENDUNG SOWIE VORRICHTUNG ZUR DURCHFÜHRUNG DES VERFAHRENS**
METHOD AND APPARATUS FOR THE RAPID SYNTHESIS OF SUBSTRATE-BOUND OR FREE PEPTIDES OR OLIGONUCLEOTIDES, FLAT MATERIAL SYNTHESIZED IN THIS WAY AND THE USE OF SUCH MATERIAL
PROCEDE ET DISPOSITIF DE SYNTHESE RAPIDE DE PEPTIDES OU D'OLIGONUCLEOTIDES LIBRES OU IMMOBILISES SUR DES SUBSTRATS, MATERIAU PLAT AINSI OBTENU ET SON UTILISATION

(30) Priorität: 31.08.1990 DE 4027675
(43) Veröffentlichungstag der Anmeldung: 10.05.1995
(73) Patentinhaber: Jerini BioTools GmbH Biochemische Produkte, 12489 Berlin (DE)
(72) Erfinder: FRANK, Ronald, D-3300 Braunschweig (DE); GÜLER, Sinan, D-3300 Braunschweig (DE)
(74) Vertreter: Boeters, Hans Dietrich, Dr.
(86) Internationale Anmeldenummer: EP9101529
(87) Internationale Veröffentlichungsnummer: WO9204366

(56) Entgegenhaltungen:
- EP-A- 0 114 599
- EP-A- 0 260 634
- Chemical Abstracts, Band 112, Nr. 21, 21. Mai 1990, Columbus,Ohio,US; siehe Seite 792, Zusammenfassung Nr. 199140e, & DD, A, 272855 (AKADEMIE DER WISSENSCHAFTEN DER DDR) 25. Oktober 1989
- Chemical Abstracts, Band 108, Nr. 19, 9. Mai 1988, Columbus, Ohio, US; siehe Seite 694, Zusammenfassung Nr. 167891b, & SU, A, 1318600 (ALL-UNION SCIENTIFIC-RESEARCH INSTITUTE OF MOLECULAR BIOLOGY) 23. Juni 1987
- Collection of Czechoslovak Chemical Communications, Band 54, Nr. 6, Juni 1989, Academia Nakladatelstvi Ceskoslovenske, Prag, TS; J. Eichler et al.: "Application of cellulose paper as support material in simultaneous solid phase peptide synthesis", Seiten 1746-1752, siehe den ganzen Artikel
- Chemical Abstracts, Band 115, Nr. 17, 1991, Columbus, Ohio, US; R. Frank et al.: "Facile and rapid "spot-synthesis" of large numbers of peptides on membrane sheets", siehe Zusammenfassung Nr. 183884b, & Pept. 1990, Proc. Eur. Pept. Symp. 21st, Meeting Date 1990, 151-2

## Beschreibung

Proteine spielen eine entscheidende Rolle in praktisch allen biologischen Prozessen wie enzymatische Katalyse, Transport und Speicherung, koordinierte Bewegung, mechanische Stützfunktion, Immunschutz, Nervenreizleitung und Kontrolle von Wachstum und Differenzierung. Proteine sind aufgebaut aus einer oder mehreren linearen Polypeptidketten. Ihre vielfältigen Funktionen bewirken sie durch dreidimensionale Faltung und Assoziation dieser Ketten. Faltung und 3D-Struktur ist determiniert durch die Aminosäureprimärsequenz der Ketten. Die Vielfalt an Proteinstrukturen in der Natur wird erzeugt durch Kombination von zwanzig Aminosäurebausteinen. Kurze Partialsequenzen (Oligopeptide) können zum gewissen Teil die lokale Struktur und Funktion einer Sequenz im Gesamtproteinverband imitieren. Oligopeptide wiederum können durch chemische Synthesen hergestellt werden. Synthetische Oligopeptide sind somit ein wichtiges Hilfsmittel bei der Strukturfunktionsanalyse von Proteinen. 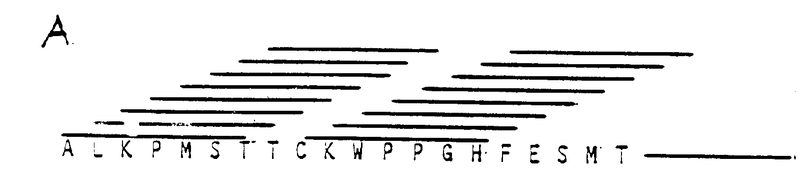
A
A L K P M S T T C K W P P G H F E S M T
 
B

A L K P M S T T R Q
T
S S
WS
W E
E F
P F
P W F
P E K
W K
P P G
H S L
 Aufgrund der Größe und Komplexität der Proteine werden für systematische Studien sehr viele Peptide gebraucht.
A) Lokalisierung einer funktionellen Region innerhalb einer längeren Proteinkette durch überlappende Peptide (vgl. M. Z. Atassi, Eur. J. Biol. 145, 1-20 (1984); H. M. Geysen, R. H. Meloen und S. J. Barteling, Proc. Natl. Acad. Sci. USA 81, 3998 (1984).
B) Entschlüsselung der für die Funktion essentieller Aminosäurereste durch Substitutionsanalyse (vgl. R. A. Houghton, Proc. Natl. Acad. Sci. USA 82, 5131 (1985); H. M. Geysen, R. H. Meloen, und S. J. Barteling, Proc. Natl. Acad. Sci. USA 81, 3998 (1984).

Biologische Testreaktionen wie enzymatische Umsetzung und Antikörperbindung sind sehr sensitiv, so daß nur geringe Mengen (ng bis µg) Peptidsubstrate ausreichen. Für eine schnelle, einfache Durchführung solcher Tests ist es vorteilhaft, daß das Peptidsubstrat an einem festen Trägermaterial immobilisiert ist und leicht aus der Reaktionslösung entfernt werden kann. Die Reaktion mit dem Peptid kann dann entweder in der verbleibenden Reaktionslösung oder durch nachfolgende Analyse des trägergebundenen Materials quantitativ vermessen werden.

Der Umfang, mit dem solche systematischen Untersuchungen durchgeführt werden können, hängt im wesentlichen von der Schnelligkeit der Peptidsynthese und von der technischen/chemischen Konzeption des Trägers ab. Schnelle Peptidsynthesen können nach dem von Merrifield entwickelten Prinzip der schrittweisen Synthese an fester Phase durchgeführt werden (R. B. Merrifield, J. Amer. Chem. Soc. 85, 2149 (1963)).

Im folgenden wird ein Verfahren beschrieben, mit dem vorteilhaft sehr viele (mehrere Hundert) immobilisierte Oligopeptide pro Arbeitstag in einer für Testzwecke ausreichenden Menge und an einem geeigneten Träger- bzw. Flachmaterial hergestellt werden können.

Dazu wird erfindungsgemäß ein Verfahren zur schnellen Synthese von trägergebundenen oder freien Peptiden vorgesehen, bei dem man von einem oder von mehreren funktionalisierten Trägern ausgeht und Schritt für Schritt entsprechend den vorgesehenen Aminosäuresequenzen die gewünschten Peptide nach einem geeigneten Syntheseverfahren synthetisiert, dadurch gekennzeichnet, daß man
- mehrere bis sehr viele verschiedene Peptide auf einem Flachmaterial gleichzeitig nebeneinander auf einander nicht berührenden, punkt- oder strichförmigen Reaktionsflächen synthetisiert, indem man
- Flachmaterial als Träger verwendet,
- je Syntheseschritt zur Kopplung kleine Aliquots der Lösungen der gewünschten Aminosäuren oder der gewünschten Di- oder Oligopeptide in Form ihrer aktivierten Derivate punkt- oder strichförmig auf den Flächenträger aufträgt,
- die Kopplungsreaktionen in den sich durch Benetzung ausbildenden Reaktionsflächen durchführt und
- gegebenenfalls die fertigen Peptide mit Hilfe einer geeigneten Abspaltungslösung vom Flächenträger abtrennt.

Gemäß einer weiteren speziellen Ausführungsform setzt man als Flachmaterial eine poröse oder nicht poröse Folie, Membran oder ein solches Papier ein, welche für die Kopplung von Aminosäuren oder Peptiden geeignete, chemisch reaktionsfähige Funktion aufweisen.

Als Trägermaterial wird ein mechanisch stabiles, gegenüber den eingesetzten Reagenzien und Lösungsmitteln chemisch inertes Flachmaterial eingesetzt. Diese kann porös oder nicht porös und nach Belieben auf einer Unterlage aufgebracht oder selbsttragend sein. Einsetzbar sind z.B. Folien, Platten, Membranen, Papiere. Wesentlich ist, daß das Flachmaterial chemisch reaktionsfähige Gruppen (Funktionen) trägt, an die die C-terminalen Bausteine für den Peptidaufbau (geeignet geschützte Aminosäuren, Di- oder Oligopeptide) kovalent verknüpft werden können.

Vorteilhafterweise ist das Trägermaterial, welches für die chemische Synthese eingesetzt wird, auch für den Einsatz in den biologischen Testreaktionen geeignet. Die hergestellten Peptide können so ohne Isolierung und nachfolgender Immobilisierung direkt eingesetzt werden, wenn die Verknüpfung zwischen Peptid und Trägermaterial so gewählt wurde, daß sie stabil gegen alle chemischen Reaktionen während der Peptidsynthese ist.

Wenn das Trägermaterial porös ist, steht auch dessen innere Oberfläche für die Synthese zur Verfügung. Waschoperationen können dann auch dadurch erfolgen, daß man Lösungen durch das Material durchsaugt. Nichtporöses Trägermaterial hat eine sehr viel geringere Oberfläche. Waschoperationen können durch einfaches Abspülen erfolgen.

Für die Peptidsynthese nach dem beanspruchten Verfahren eignen sich z. B. Flachmaterialien aus Cellulose (tragen Hydroxylfunktionen und können direkt eingesetzt werden), Glas oder Kunststoffen wie Polyethylen, Polypropylen und Teflon, and deren Oberfläche geeignete chemische Funktionen eingeführt wurden, z. B. durch Pfropfpolymerisation von Acrylsäure oder deren Derivaten. Die Funktionalisierung der Oberflächen kann ferner beispielsweise durch Einführung von Aminopropylgruppen, Glycin- oder Diglycinankergruppen erfolgen.

Die Größe der jeweiligen Reaktionsfläche hängt von dem auf dem Träger aufgebrachten Volumen, den absorbtiven Eigenschaften des Trägermaterials und der Flüchtigkeit des Lösungsmittels ab. In Abhängigkeit vom gewählten Träger, beispielsweise der spezifischen Funktionalität eines Papierträgers, wird die jeweilige Größe des Flüssigkeitsvolumens selbstverständlich auch vom gewählten Maßstab der Peptidsynthese abhängen. Die Größe des jeweiligen Flüssigkeitsvolumens bestimmt auch den minimalen Abstand zwischen zwei Reaktionsflächen und damit mittelbar die Anzahl von Reaktionsflächen, die auf einem vorgegebenen Träger vorgesehen werden können; vgl. beispielsweise Tabelle 2.

Je nach Ausführungsform und chemischer Vorbehandlung des Trägers kann die flächenspezifische Beladung mit reaktiven Funktionen in einem weiten Bereich variiert werden (z. B. 1 pmol bis 1 µmol/cm²).

Spezielle Beispiele für einsetzbare Papier sind Whatman Chrl, Whatman 540 und Whatman 3MM. Whatman 540 ist Whatman Chrl wegen seiner höheren mechanischen Stabilität vorzuziehen. Die Größe des Papierbogens als auch die Anordnung der Reaktionsflächen bzw. Flecken, bzw. Tüpfel kann frei gewählt werden. Immunologen werden das Format einer Mikrotiterplatte für 8x Tüpfel bevorzugen. In diesem Fall kann man eine Standardmikrotiterplatte wählen, bei der kleine Löcher in die Zentren der Vertiefungen eingebohrt sind und diese Standardmikrotiterplatten als Vorlage verwenden, um Papierbögen (8,5 x 12,5 cm) auszuschneiden und die Tüpfelpositionen mit einem Bleistift vorzeichnen.

Die Peptide werden dann schrittweise und entsprechend der vorbestimmten Sequenzen nach bekannten Festphasenpeptidsynthesemethoden (E. Wünsch et al. in Houben-Wevl: Methoden der organischen Chemie, 4. Auflage, Band 15 (E. Müller, Herausgeber), Thieme, Stuttgart, 1974; G. Barany, R. B. Merrifield in The Peptides, Vol. 2 (E. Gross, U. Meienhofer, Eds; Academic Press, New York, 1970, p. 1) an den Oberflächenfunktionen aufgebaut. Vorzugsweise wird nach der Fmoc/tBu-Methode verfahren (C.-D. Chang, U. Meienhofer, Int. J. Peptide Protein Res. 11, 246 (1978); E. Atherton, H. Fox, D. Harkiss, C. J. Logan, R. C. Sheppard, B. J. Williams, J. Chem. Soc. Chem. Commun. 537 (1978). Neben den zwanzig essentiellen Aminosäuren können auch beliebige modifizierte und nicht natürliche Aminosäuren eingebaut werden.

Die für den chemischen Aufbau der Peptide notwendigen Aminosäurebausteine (geeignet geschützte und aktivierte Aminosäurederivate) werden als konzentrierte Lösungen (0.1 bis 0.3 M) in einem geeigneten Lösungsmittel mit vorzugsweise hohem Siedepunkt und geringer Verdampfungsgeschwidigkeit (z. B. N-Methyl-pyrrolidinon, Kp. 203 °C) in kleinen verschließbaren Gefäßen vorgegeben.

Die Verknüpfung von Aminosäuren zu Peptiden erfolgt schrittweise, beginnend mit dem C-terminalen Ende und gegebenenfalls parallel für viele Peptide durch zyklische Ausführung von jeweils den gleichen zwei Reaktionsschritten. Die Verknüpfung sei beispielsweise folgendermaßen erläutert:
1) Peptidbindungsbildung: Ein Aliquot (0.1 bis 10 µl) der aktivierten Aminosäurelösung wird auf einen vorbestimmten Punkt oder Strich auf dem Flächenträger aufgegeben. Durch Benetzung bildet sich eine durch das aufgetragene Volumen begrenzte Reaktionsfläche (Fleck) aus. Wenn parallel viele verschiedene Peptide auf einer entsprechend großen Trägerfläche synthetisiert werden sollen, werden die Auftragsorte in einem Abstand gesetzt, daß keine überschneidung der Reaktionsflächen möglich wird. Reaktionszeit ist beispielsweise 5 bis 30 Minuten. Danach wird der Träger in einem geeigneten, verschließbaren Gefäß oder auf einer Filtriervorrichtung mehrfach beispielsweise mit Dimethylformamid (DMF) gewaschen. Die Auftragsorte können durch Markierung auf dem Träger festgelegt werden (z. B. Bleistiftmarkierungen auf Cellulosepapier). Mittels eines Farbindikators (z. B. Bromphenol Blau; V. Krchnak, J. Vagner, P. Safar, M. Lebl, Collect. Czech. Chem. Commun. 53,2542 (1988) können aber auch direkt die freien Aminofunktionen auf der Reaktionsfläche sichtbar gemacht werden, so daß eine andere Markierung nicht notwendig ist.
2) Abspaltung der N-terminalen Schutzgruppe: Der Träger wird dazu mit genügend Abspaltungslösung behandelt. Bei vorzugsweiser Durchführung der Fmoc/tBu-Methode wird die FmocSchutzgruppe mit beispielsweise 20-proz. Piperidin in DMF abgespalten. Nach beispielsweise 2 bis 10 Minuten wird mehrfach mit DMF gewaschen. Gegebenenfalls wird dann mit einer Lösung des Farbindikators behandelt, bis die Reaktionsflächen sichtbar werden. Dann wird mehrfach beispielsweise mit Ethanol gewaschen und an der Luft (gegebenenfalls mit einem Fön von max. 40 °C getrocknet.

Sind alle Peptidsequenzen fertiggestellt, werden vorzugsweise die N-terminalen Aminogruppen beispielsweise durch Acetylierung mit Essigsäureanhydrid blockiert. Dazu kann der Träger mit Acetylierungslösung (z. B. 5-proz. Essigsäureanhydrid und 5-proz. Diisopropylethylamin in DMF) behandelt und danach mehrfach mit DMF und Ethanol gewaschen werden.

Danach können die Schutzgruppen an den Aminosäureseitenketten durch geeignete Säurebehandlung (entsprechend der gewählten Synthesemethode) abgespalten werden. Dazu wird der Träger mit Abspaltungslösung behandelt. Gemäß einer speziellen Ausführungsform unter Verwendung der Fmoc/tBu-Methode (siehe die folgende Liste der verwendeten Aminosäurederivate) wird z. B. für 10 Minuten mit 50-proz. Trifluoressigsäure (TFA), 5-proz. Anisol und 1-proz. Thioanisol in Dichlormethan behandelt. Danach wird der Träger mehrfach mit Dichlormethan, dann DMF und dann Ethanol gewaschen und an der Luft getrocknet. Die Abspaltung der Schutzgruppen kann auch durch Behandeln mit 50-proz. TFA und 2-proz. Triisobutylsilan in DCM im Verlauf von etwa 60 min. erfolgen, wonach man beispielsweise mit DCM, DMF und Ethanol wäscht.

Die Peptide sind jetzt als immobilisierte Substrate für den biologischen Einsatz fertiggestellt. Hierzu kann der Träger mit den daran kovalent gebundenen Peptiden als Ganzes z. B. mit einer Testreaktionslösung behandelt werden, oder die einzelnen Reaktionsflächen können durch Zerschneiden des Blattes getrennt und individuell behandelt werden.

Der einfachste Weg, Peptide auf die beschriebene Weise an Trägern aufzubauen ist der, die entsprechenden C-terminalen Aminosäurebausteine direkt mit den reaktiven Funktionen auf der Trägeroberfläche über stabile Amid- oder Esterbindungen zu verknüpfen. Gemäß spezielleren Ausführungsformen kann zunächst ein sog. bifunktionelles Linkerreagenz (vgl. E. Atherton et al. J. Chem. Soc. Perkin Trans. I 538-546 (1981)) an die Trägerfunktionen geknüpft werden. Das wiederum daran aufgebaute Peptid kann dann entsprechend der chemischen Natur der Peptid-Linker-Bindung z. B. unter geeigneten Reaktionsbedingungen mit C-terminaler Carboxyl- oder Carboxamidgruppe abgespalten werden und in löslicher Form für Testzwecke eingesetzt werden (labiler Linker!). Das Linkerreagenz kann aber auch als ein Abstandshalter (Spacer) eingesetzt werden, um die Zugänglichkeit des Peptids für große Reaktionspartner wie Antikörper oder Enzyme zu erhöhen (stabiler Linker!).

Bei der Verwendung von Papierträgern kann man zweistufig vorgehen.

Erste Stufe (A): Es wird eine gleichmäßige Verteilung von reaktiveren Aminofunktionen vorgesehen. Dazu werden üblicherweise die Hydroxylgruppen der Cellulose mit einem N-Fmoc-geschützten Aminosäurederivat verestert und anschließend durch Behandlung mit 20-proz. Piperidinlösung in DMF die Aminogruppen freigesetzt.

Zweite Stufe (B): Es wird eine weitere N-Fmoc-Aminosäure an die eingeführten Aminofunktionen punkt- oder strichförmig angehängt. Danach werden alle restlichen Aminofunktionen durch Acetylierung blockiert und Fmoc abgespalten.

Im Rahmen der vorliegenden Erfindung wurde auch Fmoc-Glycin für beide Derivatisierungsstufen verwendet. Jedoch wurden nicht unbeträchtliche Mengen des Gly-Gly-Anker bei der Fmoc-Entschützung und bei längerer Lagerung verloren, vermutlich in Folge einer Diketopiperazinbildung. Ferner scheint das Gly-Gly-Ende der Peptide die Antikörperbindung zu stören, auch scheinen einige Sera unspezifisch an Ac-Gly-Gly zu binden. Fmoc-β-Ala ist die bessere Wahl. β-Alanin ist eine unübliche Aminosäure, die in natürlichen Proteinen nicht vorliegt, wobei der β-Ala-β-Ala-Anker keiner Diketopiperazinbildung unterliegt und der Abstandshalter vergrößert wird. Getrocknete Flachmaterialien, die mit Flecken von β-Ala-β-Ala derivatisiert und mit BPB angefärbt worden waren, zeigten nach mehrmonatiger Lagerung bei - 20 °C keinen Verlust an Aminosäurefunktionen. Für längere Lagerungen bei Temperaturen bis zu 30 °C ist ein Fmoc-geschützter Anker noch stabiler. Zur Erläuterung sei auf **Figur 6** verwiesen. Diese Figur zeigt eine typische Anordnung von Flecken auf einem Papierblatt (β Ala-β-Ala-Anker mit BPB angefärbt). Das Format war dabei einer für 96 Flecken vorgesehenen Mikrotiterplatte angepaßt.

Das vorgestellte Verfahren weist gegenüber dem Stand der Technik (Geysen et al. 1984; Frank und Krause, Deutsche Patentanmeldung P 39 35 572.1) folgende Vorteile auf:
- äußerst einfache Handhabung; für die chemischen Kopplungen der Aminosäurederivate zu Peptiden müssen nur kleine Volumina der aktivierten Aminosäurelösungen auf den Flächenträger aufgetüpfelt werden;
- noch kleinere Reaktionsvolumina sind möglich, z. B. 0.1 bis 1 µl, wodurch ein noch sparsamerer Verbrauch an den teuren Aminosäurederivaten erzielt wird;
- die Träger wie Cellulose- oder Glasfaserpapiere sind leicht zugänglich oder im Handel zu beziehen;
- der Synthesemaßstab einzelner Peptide kann wegen der variablen flächenspezifischen Beladung mit reaktiven Funktionen innerhalb eines weiten Bereichs gewählt werden;
- die Pipettierschritte (insbesondere bei automatischer Durchführung) können extrem schnell ausgeführt werden, so daß noch mehr Peptide pro Zeiteinheit hergestellt werden können.

Das vorstehend beschriebene Verfahren kann in entsprechender Weise auch zur schnellen Synthese von trägergebundenen oder freien Oligonucleotiden herangezogen werden. Zum Chemismus und zur Abwicklung von Oligonucleotidsynthesen kann bespielsweise auf das europäische Patent 84 100 059.9 verwiesen werden.

Das vorliegend beschriebene Verfahren kann auch automatisiert werden. Hierzu können für das Aliquotieren und Auftüpfeln der aktivierten Aminosäurelösungen bzw. Nucleotidlösungen sowie das Aufgeben der anderen Wasch- und Reaktionslösungen Pipettierautomaten eingesetzt werden. Der Flächenträger muß dann auf einer geeigneten Wasch- oder Filtriervorrichtung montiert werden.

Im Handel erhältliche x/y-programmierbare TLC-Spotter oder Pipetten-Arbeitsstationen sind geeignete Vorrichtungen zur automatischen Durchführung des erfindungsgemäßen Verfahrens. Volumina bis herab zu 10 nl können präzise an jeder gewünschten Stelle aufgetragen werden, wobei mehrere tausend Peptide auf einem gut handhabbaren Flachmaterialträger synthetisiert werden können. Damit liegt man 2 Größenordnungen unter der litographischen Technik von Fodor et al., Sience, 251, 767-773 (1991). Selbst in den kleinsten Flüssigkeitsmengen (etwa 10 nl) wird jedes Peptid in nmol-Mengen gebildet, die zur Isolation und erschöpfenden Charakterisierung ausreichen.

### Ausführungsbeispiel 1:

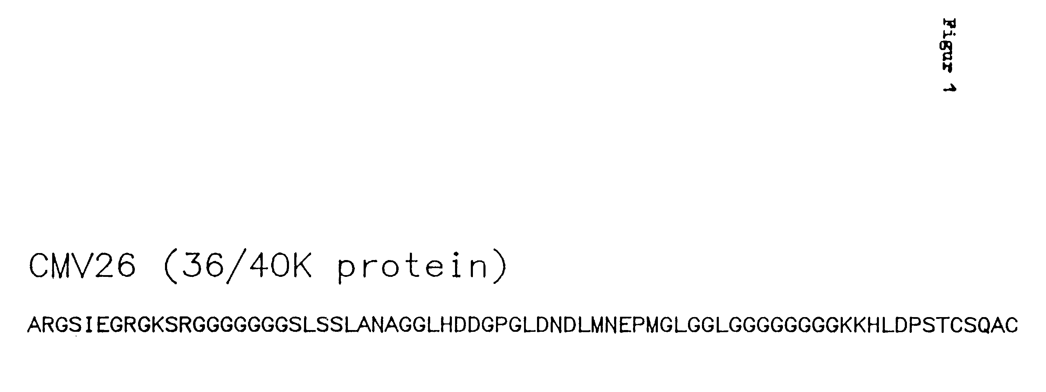

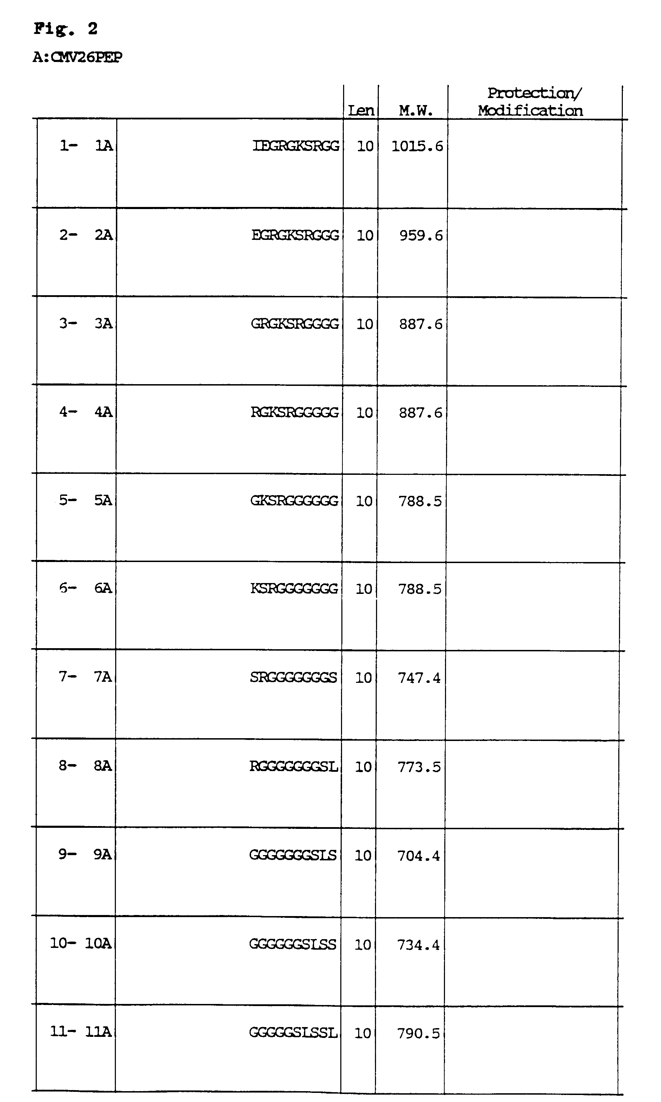

Analyse eines immunogenen Teilabschnittes (CMV26) des 36/40K-Proteins vom Cytomegalie-Virus. **Figur 1:** Aminosäureprimärsequenz des klonierten, immunogenen Teilabschnittes CMV26. Davon abgeleitet wurden überlappende, um jeweils eine Aminosäure verschobene Dekapeptidsequenzen. **Figuren 2 A - C:** Liste aller zu synthetisierenden Dekapeptide mit der zugeordneten Nummerierung und Vorschrift für die simultane, parallele Durchführung der Aminosäurekopplungen.

Flächenförmiger Träger: Ein 5,5 x 10,5 cm großes Blatt Cellulosepapier (Chrl oder 540, Whatman, England) wurde ausgeschnitten. Danach wurden 50 Punkte im Abstand von 1 cm mit Bleistift darauf markiert. Das Blatt wurde in ein zylinderförmiges, mit Schliffstopfen verschließbares Glasgefäß gegeben und mit 1 ml einer Lösung von
0,2 M Fmoc-Glycin
0,3 M Hydroxybenzotriazol (HOBt)
0,24 M Diisopropylcabodiimid (DIC)
0,2 M N-Methylimidazol (MeIm) in DMF
für 3 Stunden bei Raumtemperatur behandelt, anschließend dreimal mit je 10 ml DMF gewaschen, dann für 10 Minuten mit 20-proz. Piperidin in DMF behandelt (Abspaltung der Fmoc-Gruppe), danach dreimal mit je 10 ml DMF und zweimal mit je 10 ml Ethanol (abs) gewaschen und mit einem Fön getrocknet. Die quantitative Bestimmung der so an die Hydroxylgruppen des Cellulosepapiers veresterten Menge an Glycin (freie Aminogruppen) ergab 0.2 bis 0.3 µmol/cm².

Das Blatt wurde in eine flache Schale aus Glas oder Polyethylen gelegt. Dann wurden je Aliquots (1 µl) einer Lösung von 0.3 M Fmoc-Glycin, 0,45 M HOBt, 0.36 M DICD in N-Methyl-pyrrnlidinon (MPD) auf die vormarkierten Punkte des Papiers aufgetragen und die Schale mit einer Glasscheibe zugedeckt. Nach 20 Minuten wurde das Blatt in den Glaszylinder gegeben und dreimal mit je 10 ml DMF gewaschen. Dann wurden alle noch auf dem Papier vorhandenen freien Aminogruppen der ersten Glycinkopplung durch Acetylierung blockiert. Dazu wurde für 10 Minuten mit 3 ml 5-proz. Essigsäureanhydrid und 5-proz. Diisopropylethylamin in DMF behandelt. Anschließend wurde dreimal mit je 10 ml DMF gewaschen, dann mit 20-proz. Piperidin in DMF behandelt und wieder dreimal mit DMF gewaschen. Danach wurden 3 ml 0.3-proz. Bromphenol Blau (BPB) in DMF zugegeben, wodurch die Reaktionsflächen der 2. Glycinkopplung blau anfärben. Es wurde dann zweimal mit je 10 ml Ethanol gewaschen und getrocknet.

Der so vorbehandelte Celluloseflächenträger bietet an den markierten Reaktionsflächen Diglycinankergruppen mit freien primären Aminofunktionen für den Aufbau von Peptiden an. Die 49 in **Figur 2A** aufgeführten Dekapeptide wurden fortlaufend nummeriert und an den entsprechend nummerierten Reaktionsflächen wie voranstehend beschrieben simultan-parallel synthetisiert. Die Verteilung der aktivierten Aminosäurelösungen wurde anhand der in **Figur 2B** und **2C** gezeigten Liste ausgeführt.

### Synthesemethode: Fmoc/tBu-Methode

Verwendete Aminosäurederivate:
Fmoc Ala
Fmoc-Arg(Pmc)
Fmoc-Asn(Tmob)
Fmoc-Asp(OtBu)
Fmoc-Cys(Butthio)
Fmoc-Gln(Tmob)
Fmoc-Glu(OtBu)
Fmoc-Gly
Fmoc-His(Boc)-Pfp
Fmoc-Ile
Fmoc-Leu
Fmoc-Lys(Boc)
Fmoc-Met
Fmoc-Phe
Fmoc-Pro
Fmoc-Ser(tBu)
Fmoc-Thr(tBu)
Fmoc-Trp
Fmoc-Tyr(tBu)
Fmoc-Val

Alle Schutzgruppen der Aminosäureseitenketten (-) mit Ausnahme von (Butthio) an Cys können mit 20- bis 50-proz. TFA in Dichlormethan abgespalten werden. Die Butthio-Gruppe kann dann anschließend mit z. B. Dithiothreitol (DTT) oder Dithioerythritol (DTE) entfernt werden.

Die Aktivierung der Aminosäurederivate kann nach Standardmethoden erfolgen, z. B. durch 1,5-fachen Überschuß von 1-Hydroxybenzotriazol (HOBt) und 1,2-fachen Überschuß an Diisopropylcarbodiimid (DIC) in N-Methylpyrrolidinon für eine Stunde. Andere Aktivierungsmethoden beispielsweise mit symmetrischen Anhydriden, Pentafluorphenolester oder BOP sind ebenso möglich. Entsprechend dem vorausberechneten Verbrauch wurden 0.2 bis 1 ml Lösungen angesetzt. Es wurden Aliquots von 1 µl aufgetragen. Die mit BPB blau gefärbten Reaktionsflächen zeigten nach 5 bis 20 Minuten einen Farbumschlag nach Gelb, was einen vollständigen Umsatz der freien Aminogruppen anzeigte. Abspaltung der Fmoc-Schutzgruppe wurde mit 20-proz. Piperidin in DMF über 5 Minuten durchgeführt. Endacetylierung der fertigen Peptide wurde durch Behandlung mit 5-proz. Essigsäureanhydrid, 5-proz. Diisopropylethylamin in DMF über 10 Minuten erreicht. Die Schutzgruppen der Aminosäureseitenketten wurden mit 20-proz. TFA und 1-proz. Triisobutylsilan in Dichlormethan über 20 Minuten abgespalten und der Träger anschließend mit je 10 ml Portionen dreimal mit Dichlormethan, dreimal mit DMF und dreimal mit Ethanol gewaschen und getrocknet.

Mit diesen Peptiden wurde z. B. die sequenzspezifiscne Bindung von anti-CMV26 Antikörpern in einem Kaninchenserum durch einen Farbtest nachgewiesen.

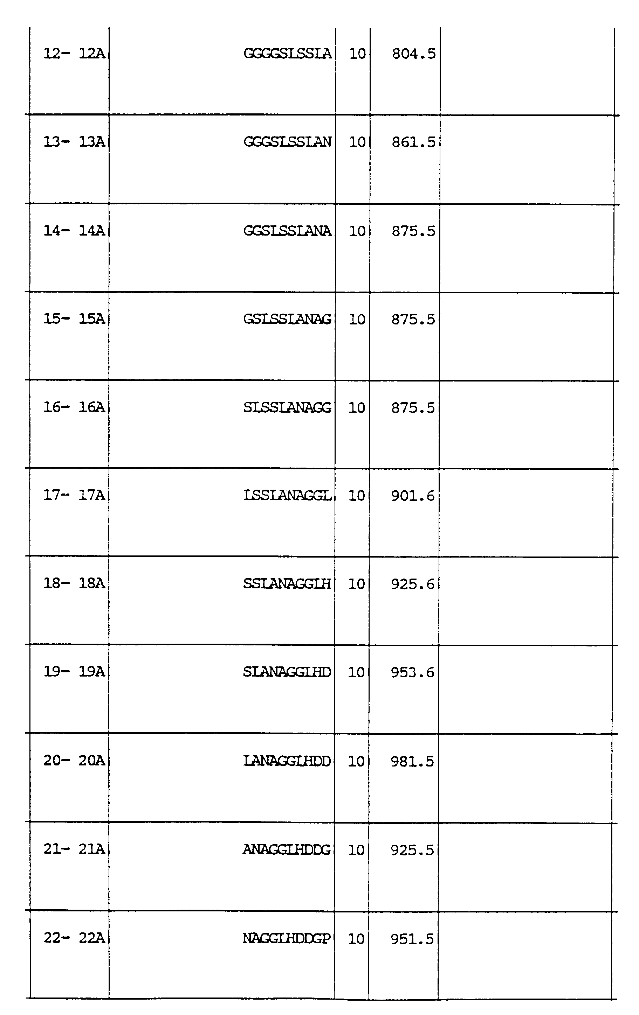

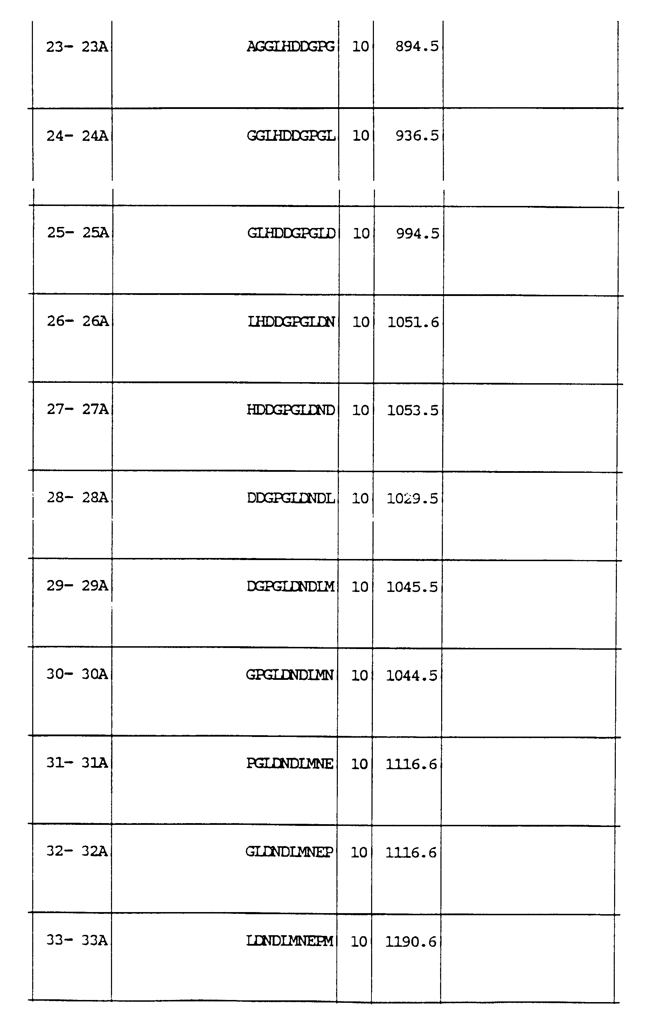

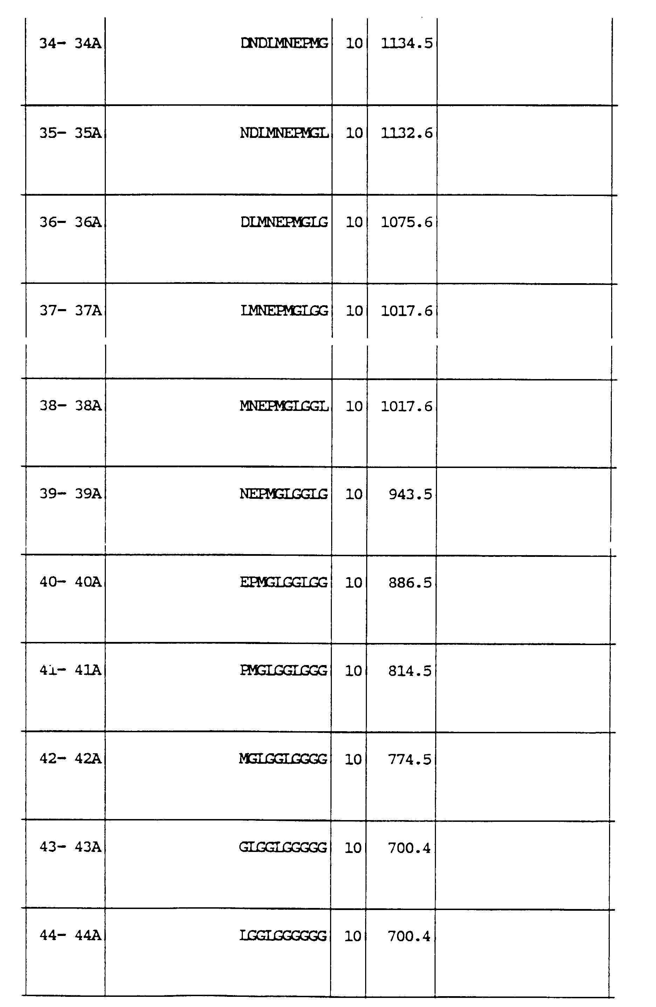

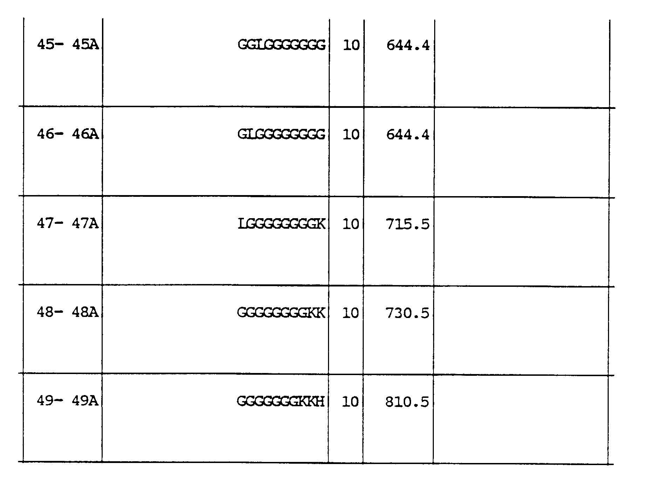

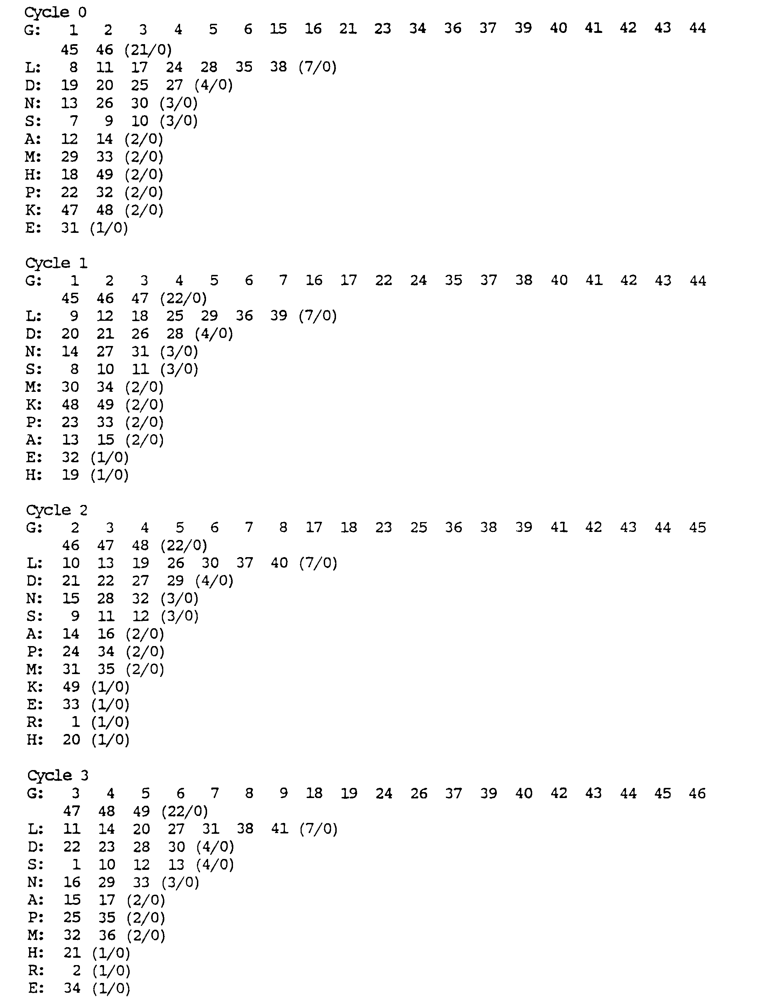

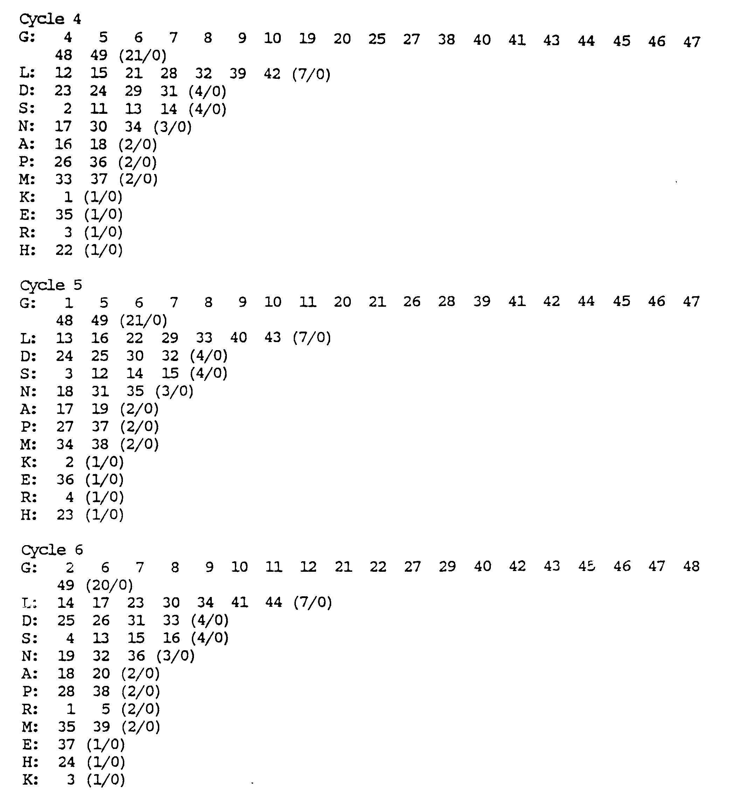

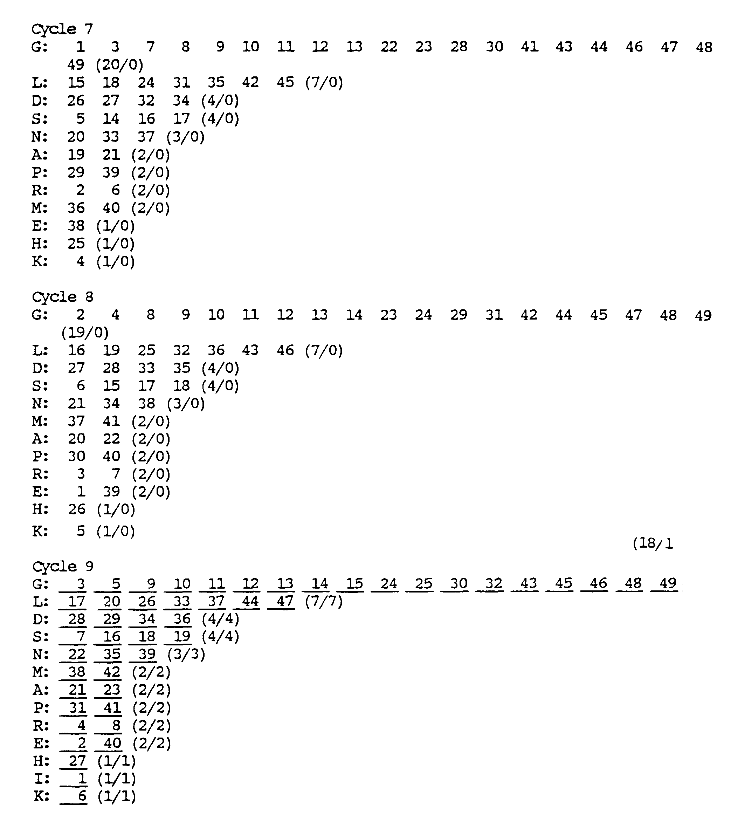

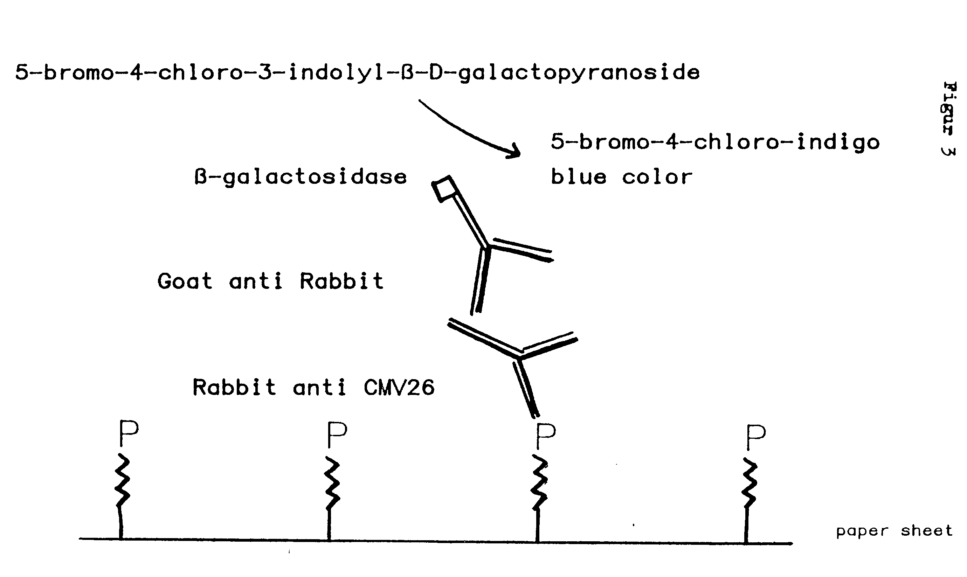

**Figur 3:** Schematische Darstellung des Farbtests. Die oberflächengebundenen Peptide (P) werden mit dem Kaninchen-anti-CMV26-Serum inkubiert, überschüssiges Serum abgewaschen, und danach mit einem anti-Kaninchen-Antikörper aus der Ziege, welcher kovalent mit dem Enzym β-Galactosidase verknüpft ist, inkubiert. Nur wenn die anti-CMV26-Antikörper das Peptid binden können, wird über den zweiten Antikörper das Enzym gebunden. Dieses kann dann durch seine Aktivität bei der Spaltung von 5-Brom-4-chlor-3-indolyl-β-D-galactopyranosid nachgewiesen werden, wobei sich in Gegenwart eines milden Oxidationsmittels wie Kaliumhexacyanoferrat(III) ein in Wasser und Alkohol unlöslicher blauer Indigofarbstoff bildet.

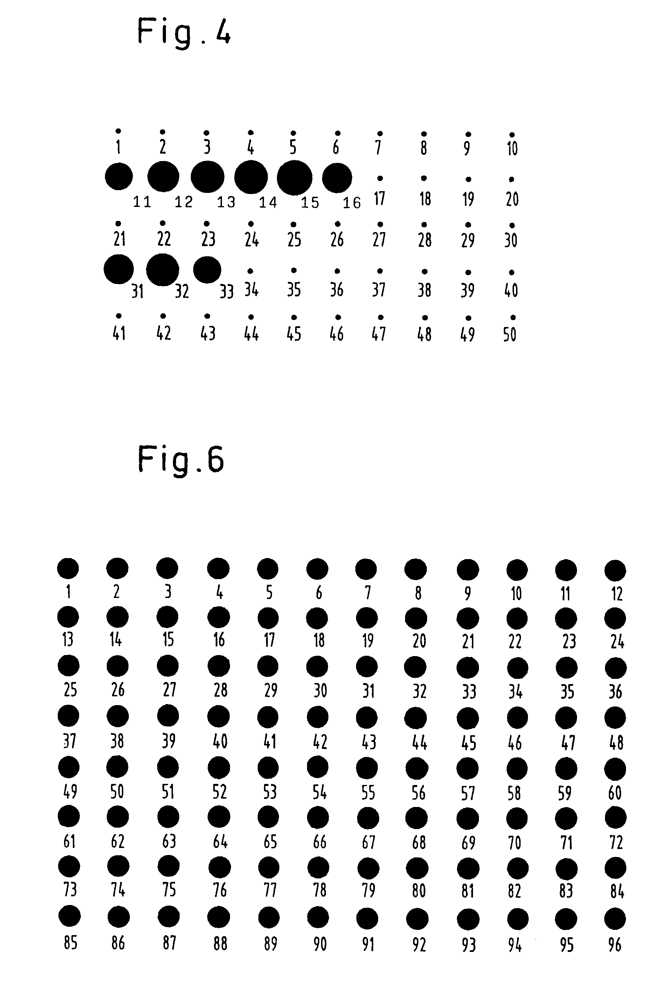

**Figur 4:** Fotographie des flachen Celluloseträgers nach dem Farbtest. Die Peptide an den Reaktionsflächen 11 bis 16 und 31 bis 33 reagieren positiv.

Durch Behandlung des Trägers mit Detergenzien (wie Harnstoff, Natriumdodecylsulfat und Mercaptoethanol) können die gebundenen Antikörper wieder abgelöst werden. Der Indigofarbstoff kann mit DMF abgewaschen werden. Danach kann der Träger mit den immobilisierten Peptiden erneut eingesetzt werden. Dies kann mehrfach wiederholt werden, solange keine Reaktionsbedingungen angewandt werden, welche die Peptide ablösen oder zerstören. Nach mehr als zehn Antikörperbindungstests mit dem gleichen Träger wurden einige Reaktionsflächen ausgeschnitten und die Peptide mit 1M NaOH abgelöst. Die CMV26-Gly-Gly-Peptide ließen sich in der HPLC-Analyse als auch durch FAB-Massenspektroskopie eindeutig nachweisen.

### Ausführungsbeispiel 2:

Analyse des gleichen immunogenen Teilabschnittes (CMV26) des 36/40K-Proteins vom Cytomegalie-Virus.
Flächenträger: Ein 5,5 x 10,5 cm großes Blatt Glasfaserpapier (QM-A, Whatman, England) wurde ausgeschnitten; danach wurden 50 Punkte im Abstand von je 1 cm mit Bleistift darauf markiert. Das Blatt wurde in einem Erlenmeyerkolben (250 ml) mit Schliff mit 50 ml 2-proz. Aminopropyl-triethoxysilan in abs. Toluol über Nacht im Rückfluß gekocht. Anschließend wurde jeweils mit Portionen von 10 ml dreimal mit Toluol und dreimal mit Aceton gewaschen und 1 Stunde bei 110 °C getrocknet. Die quantitative Bestimmung der so eingeführten Aminopropylgruppen (freie Aminogruppen) ergab 30 bis 50 nmol/cm².

Alle weiteren chemischen Operationen wurden genauso durchgeführt, wie im Ausführungsbeispiel 1 für Cellulosepapier nach der ersten Glycinkopplung beschrieben wurde. Das Ergebnis des Antikörperbindungstests war identisch.

Die Ausführungsbeispiele 1 und 2 belegen einen wichtigen Anwendungsaspekt für die durch das beanspruchte Verfahren hergestellten Flachmaterialien mit den daran gebundenen Peptiden. Sie können für diagnostische Zwecke in der Human- und Veterinärmedizin zum sehr spezifischen Nachweis von bakteriellen und viralen Infektionen eingesetzt werden. Solche Infektionen führen durch Immunreaktionen des befallenen Organismus zur Bildung von Antikörpern gegen virale oder bakterielle Proteine. Mit virus- oder bakterienspezifischen Peptiden können solche Antikörper im Serum nachgewiesen und damit auch die Art und Subklasse des Erregers bestimmt werden. Für das Auffinden der speizifischen Peptide kann wiederum das gleiche Verfahren eingesetzt werden, indem (wie in den Ausführungsbeispielen gezeigt) Serien von an Flachmaterial gebundenen Peptiden hergestellt und getestet werden.

### Ausführungsbeispiel 3:

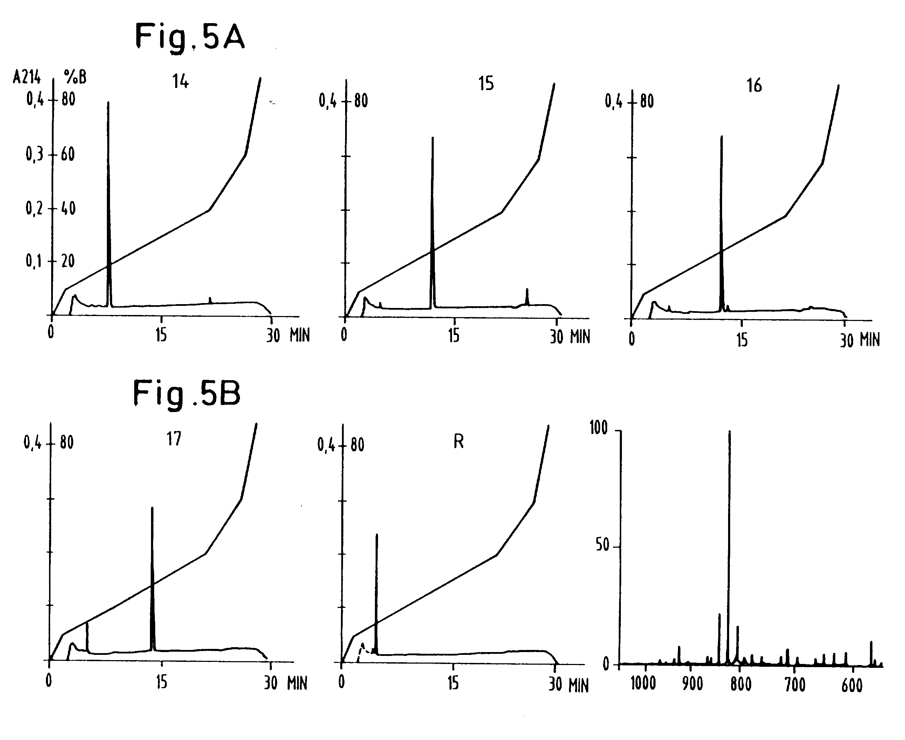

Es wurden vier zu CMV26 in Beziehung stehende Oligopeptide durch punktförmige Synthese auf Papierträgern mit Boc-Lys-Pro-Derivatisierung synthetisiert und von den Trägern mit Hilfe von Diketopiperazin-bildung abgespalten. Einzelheiten sind **Figur 5 A-B und Tabelle 1** zu entnehmen. Es zeigen:
- **Figur 5 A:**: HPLC-Spuren der rohen Peptide;
- **Figur 5 B:**: FAB-Massenspektrum von Peptid 15 (auf Basis positiver Ionen);

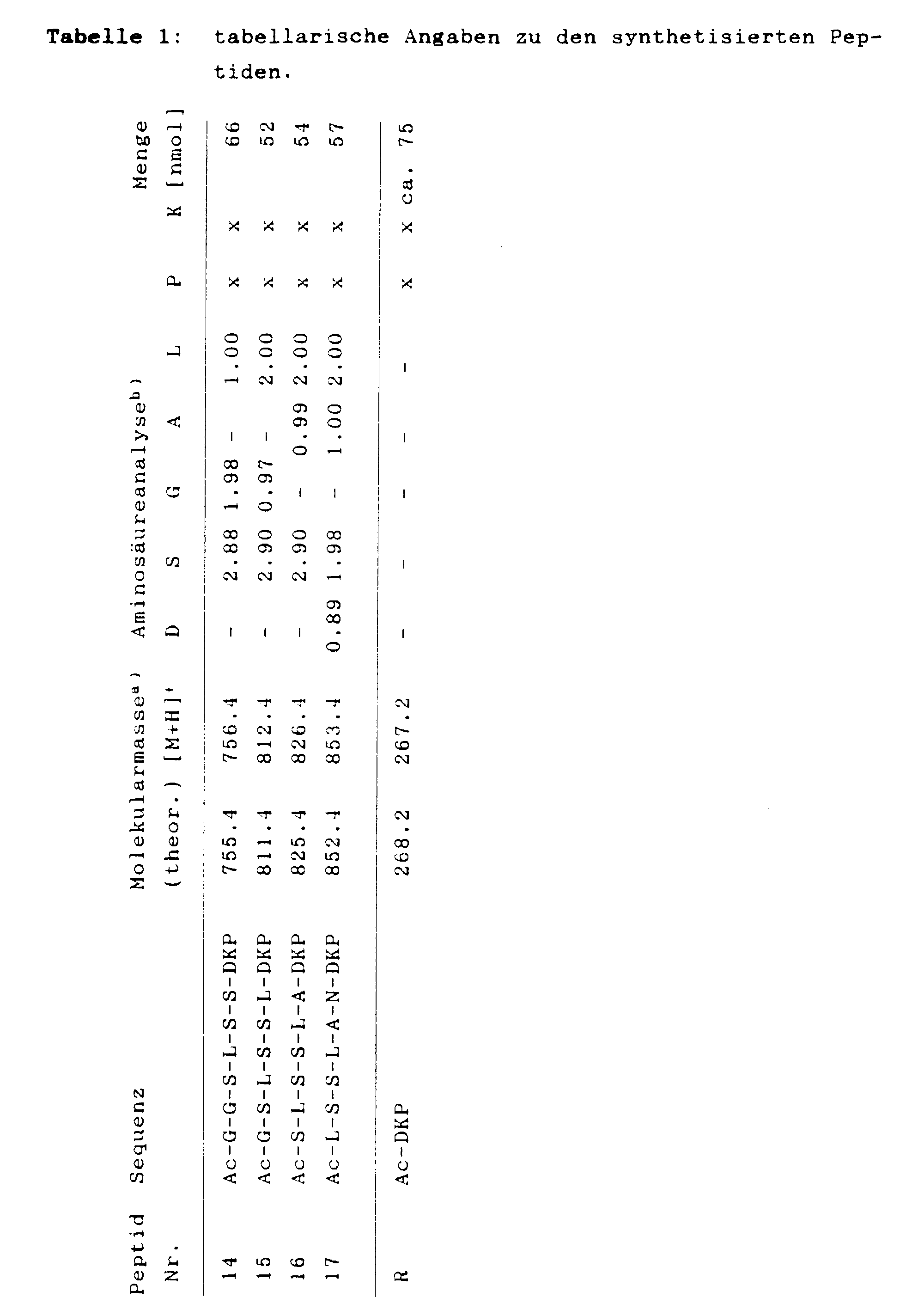

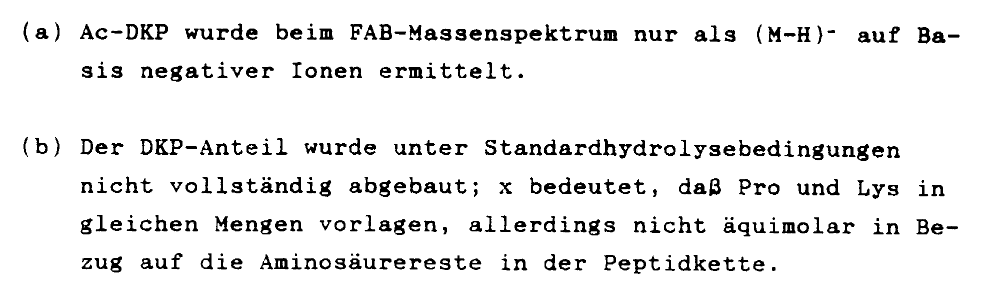

### Ausführungsbeispiel 4:

Es wurde von Hand mit Volumina bis herunter zu 0,1 µl mit einer gewöhnlichen Mikropipette getüpfelt, die mit einer Polypropylenspitze versehen war. Einige typische Daten, die bei der Verwendung von zwei verschiedenen Papierqualitäten ermittelt wurden, sind in der folgenden **Tabelle 2** zusammengestellt.

**Tabelle 2:**

| Beziehung zwischen Tüpfelvolumen, Fleckengröße und Funktionalität. | | | | | |
|---|---|---|---|---|---|
| A: Whatmann 540 | | β-ala-Anker | B: Whatman 3 MM vorbehandelt | | β-ala-Anker |
| Volumen [µl] | Durchmesser [mm] | Anker [µmol] | Volumen [µl] | Durchmesser [mm] | Anker [µmol] |
| 0.1 | 3 | 0.02 | 1 | 4 | 0.10 |
| 0.5 | 5 | 0.07 | 5 | 7 | 0.45 |
| 1.0 | 7 | 0.13 | 10 | 10 | 0.85 |
| 2.0 | 9 | 0.25 | 20 | 14 | 1.70 |
| 5.0 | 12 | 0.60 | 50 | 22 | 4.40 |
| Werte sind ± 5% | | | | | |

### Ausführungsbeispiel 5:

Es werden alle 16 Dinucleosidmonophosphate der allgemeinen Formel N₂pN₁ hergestellt, wobei man sich hinsichtlich des Chemismus an das europäische Patent 84 100 059.9 sowie Frank et al. in Nucleic Acids Res., 11 (1983) 4365-4377 und für den verwendeten Träger an Ausführungsbeispiel 1 anlehnt, wobei noch auf folgende Einzelheiten hingewiesen werden soll.

Als flächenförmigen Träger verwendet man Whatman 540-Papier mit Di-β-Ala-Ankern gemäß Ausführungsbeispiel 1. Der erste Startbaustein N₁ wird jeweils als 5'-DMTr-Nucleosid-3'-succinat eingesetzt (in NMP) und in Analogie zu Ausführungsbeispiel 1 durch Tüpfeln an die Aminoanker gekoppelt (mit DIC und HOBt). Die Succinate sind ein Beispiel für einen labilen Linker. Danach wird ein aus dem angeführten Stand der Technik bekannter Capping-Schritt vorgenommen. Die Abspaltung der DMTr-Gruppe kann mit 3-proz. Dichloressigsäure in Dichlormethan erfolgen. Zur Kopplung wird der zweite Baustein N₂p als 5'-DMTr-Nucleosid-3'-o-Chlorphenylphosphat (mit MSNT in Pyridin) unter einem Argonkissen aufgetüpfelt, um Luftfeuchtigkeit auszuschließen. Die DMTr-Gruppe wird wie vorstehend angegeben abgespalten.

Die Flecken werden ausgestanzt, jeweils mit 30-proz. wässerigem Amoniak behandelt und die Überstände auf ihre Reaktionsprodukte (Dinucleosidmonophosphate) durch FAB-Massenspektroskopie gemäß Grotjahn et al in Nucleic Acids Res., 10 (1982) 4671-4678 untersucht.

## Patentansprüche

1. Verfahren zur schnellen Synthese von trägergebundenen oder freien Peptiden, bei dem man von einem oder von mehreren funktionalisierten Trägern ausgeht und Schritt für Schritt entsprechend den vorgegebenen Aminosäuresequenzen die gewünschten Peptide nach einem geeigneten Syntheseverfahren synthetisiert, dadurch **gekennzeichnet,** daß man
- mehrere bis sehr viele verschiedene Peptide auf einem Flachmaterial gleichzeitig nebeneinander auf einander nicht berührenden punkt- oder strichförmigen Reaktionsflächen synthetisiert, indem man
- Flachmaterial als Träger verwendet,
- je Syntheseschnitt zur Kopplung kleine Aliquots der Lösungen der gewünschten Aminosäuren oder der gewünschten Di- oder Oligopeptide in Form ihrer aktivierten Derivate punkt- oder strichförmig auf den Flächenträger aufträgt,
- die Kopplungsreaktionen in den sich durch Benetzung ausbildenden Reaktionsflächen durchführt und
- gegebenenfalls die fertigen Peptide mit Hilfe einer geeigneten Abspaltungslösung vom Flächenträger abtrennt.

2. Verfahren nach Anspruch 1, dadurch **gekennzeichnet,** daß als Flachmaterial eine poröse oder nicht poröse Folie, Membran oder ein solches Papier eingesetzt wird, welche für die Kopplung von Aminosäuren oder Peptiden geeignete, chemisch reaktionsfähige Funktionen aufweisen.

3. Verfahren nach Anspruch 2, dadurch **gekennzeichnet,** daß das Flachmaterial aus Cellulose, Glas oder organischen Polymeren besteht und seine Oberfläche geeignete chemische Funktionen aufweist.

4. Verfahren nach Anspruch 2 oder 3, dadurch **gekennzeichnet,** daß die
Funktionalisierung der Oberflächen durch Einführung von Aminopropylgruppen, Glycin- oder Diglycinankergruppen erfolgt.

5. Verfahren nach Anspruch 3 oder 4, dadurch **gekennzeichnet,** daß man
die zu synthetisierenden Peptide (a) C-terminal direkt mit dem Flachmaterial verknüpft oder (b) mit dem Flachmaterial über einen Linker verknüpft aufbaut.

6. Verfahren nach einem der vorhergehenden Ansprüche, dadurch **gekennzeichnet,** daß die flächenspezifische Beladung mit reaktiven Funktionen 1 pmol bis 1 µmol pro cm² beträgt.

7. Verfahren zur schnellen Synthese von trägergebundenen oder freien Oligonucleotiden, bei dem man von einem oder von mehreren funktionalisierten Trägern ausgeht und Schritt für Schritt entsprechend den vorgegebenen Nucleotidsequenzen die gewünschten Oligonucleotide nach einem geeigneten Syntheseverfahren synthetisiert, dadurch **gekennzeichnet,** daß man
- mehrere bis sehr viele verschiedene Oligonucleotide auf einem Flachmaterial gleichzeitig nebeneinander auf einander nicht berührenden, punkt- oder strichförmigen Reaktionsflächen synthetisiert, indem man
- Flachmaterial als Träger verwendet
- je Syntheseschnitt zur Kopplung kleine Aliquots der Lösungen der gewünschten Nucleotide oder der gewünschten Di- oder Oligonucleotide in Form ihrer aktivierten Derivate punkt- oder strichförmig auf den Flächenträger aufträgt,
- die Kopplungsreaktion in den sich durch Benetzung ausbildenden Reaktionsflächen durchführt und
- gegebenenfalls die fertigen Oligonucleotide mit Hilfe einer geeigneten Abspaltungslösung vom Flächenträger abtrennt.

8. Verfahren nach Anspruch 7, dadurch **gekennzeichnet,** daß als Flachmaterial eine poröse oder nicht poröse Folie, Membran oder ein solches Papier eingesetzt wird, welche für die Kopplung von Nucleotiden geeignete, chemisch reaktionsfähige Funktionen aufweisen.

9. Verfahren nach Anspruch 7 oder 8, dadurch **gekennzeichnet,** daß man sich der Maßnahmen von Anspruch 3, 4 oder 6 bedient.

10. Verfahren nach Anspruch 9, dadurch **gekennzeichnet,** daß man die zu synthetisierenden Oligonucleotide (a) 3'-terminal direkt mit dem Flachmaterial verknüpft oder (b) mit dem Flachmaterial über einen Linker verknüpft aufbaut.

11. Flachmaterial mit daran gebundenen Peptiden, hergestellt nach einem der Ansprüche 1 bis 6.

12. Flachmaterial mit daran gebundenen Oligonucleotiden, hergestellt nach einem der Ansprüche 7 bis 10.

13. Verwendung des Flachmaterials nach Anspruch 11 für mikrobiologische und insbesondere diagnostische Zwecke in der Human- und Veterinärmedizin zum spezifischen Nachweis von bakteriellen und viralen Infektionen.

14. Verwendung des Flachmaterials nach Anspruch 12 für Hybridisierungszwecke.

## Claims

1. Process for the rapid synthesis of support-bound or free peptides, starting from one or more functionalized supports and synthesizing the required peptides step by step corresponding to the given amino acid sequences according to a suitable synthesis procedure, **characterized** in that
- several to very many different peptides are synthesized simultaneously alongside each other on a flat material on spot-shaped or linear reaction areas which are not in contact with each other,
- using flat material as the support,
- at each synthesis step, for the coupling, transferring small aliquots, in the shape of spots or lines, of solutions of the required amino acids or of the required di- or oligopeptides, in the form of their activated derivatives, to the planar support,
- carrying out the coupling reactions in the reaction areas, which develop by wetting, and,
- where appropriate, separating the completed peptides from the planar support using a suitable cleaving solution.

2. Process according to claim 1, **characterized** in that the flat material employed is a porous or non-porous film, membrane, or a paper of this nature, which possesses a chemically reactive functionality which is suitable for coupling amino acids or peptides.

3. Process according to claim 2, **characterized** in that the flat material is composed of cellulose, glass or organic polymers and its surface possesses a suitable chemical functionality.

4. Process according to claim 2 or 3, **characterized** in that the functionalization of the surfaces takes place by the introduction of aminopropyl groups or glycine or diglycine anchor groups.

5. Process according to claim 3 or 4, **characterized** in that the peptides being synthesized are constructed (a) linked directly by their C-terminus to the flat material or (b) linked to the flat material via a linker.

6. Process according to one of the preceding claims, **characterized** in that the surface-specific loading with reactive functionalities is from 1 pmol to 1 pmol per cm².

7. Process for the rapid synthesis of support-bound or free oligonucleotides, starting from one or more functionalized supports and synthesizing the required oligonucleotides step by step corresponding to the given nucleotide sequences according to a suitable synthesis procedure, **characterized** in that
- several to very many different oligonucleotides are synthesized simultaneously alongside each other on a flat material on spot-shaped or linear reaction areas which are not in contact with each other,
- using flat material as the support,
- at each synthesis step, for the coupling, transferring small aliquots, in the shape of spots or lines, of solutions of the required nucleotides or of the required di- or oligonucleotides, in the form of their activated derivatives, to the planar support,
- carrying out the coupling reaction in the reaction areas, which develop by wetting, and,
- where appropriate, separating the completed oligonucleotides from the planar support using a suitable cleaving solution.

8. Process according to claim 7, **characterized** in that the flat material employed is a porous or non-porous film, membrane, or a paper of this nature, which possesses a chemically reactive functionality which is suitable for coupling nucleotides.

9. Process according to claim 7 or 8, **characterized** in that the measures of claim 3, 4 or 6 are used.

10. Process according to claim 9, **characterized** in that the oligonucleotides being synthesized are constructed (a) linked directly by their 3'-terminus to the flat material or (b) linked to the flat material via a linker.

11. Flat material with peptides bound to it, prepared according to one of claims 1 to 6.

12. Flat material with oligonucleotides bound to it, prepared according to one of claims 7 to 10.

13. Use of the flat material according to claim 11 for microbiological and, in particular, diagnostic purposes in human and veterinary medicine for the specific detection of bacterial and viral infections.

14. Use of the flat material according to claim 12 for hybridization purposes.

## Revendications

1. Procédé pour la synthèse rapide de peptides immobilisés sur des substrats ou libres, dans lequel on part d'un ou plusieurs substrats fonctionnalisés et on synthétise les peptides souhaités par étapes, conformément aux séquences d'acides aminés prédéfinies, selon un procédé de synthèse approprié, caractérisé en ce qu'on
- synthétise simultanément, en parallèle, d'une pluralité à une multitude de peptides différents, sur des surfaces de réaction en forme de points ou de stries n'entrant pas en contact les unes avec les autres,
- utilise un matériau plat comme support,
- applique à chaque étape de synthèse, pour le couplage, de petites quantités aliquotes des solutions des acides aminés souhaités ou des di- ou oligopeptides souhaités sous la forme de leurs dérivés activés, sous forme de points ou de stries sur les supports plats,
- effectue les réactions de couplage dans les surfaces réactionnelles qui se forment par imprégnation et
- sépare éventuellement du support plat les peptides formés, au moyen d'une solution de séparation appropriée.

2. Procédé selon la revendication 1, caractérisé en ce qu'on utilise comme matériau plat une feuille ou une membrane poreuse ou non poreuse ou un papier, présentant des fonctions ayant une réactivité chimique, appropriées pour le couplage des acides aminés ou des peptides.

3. Procédé selon la revendication 2, caractérisé en ce que le matériau plat consiste en de la cellulose, du verre ou des polymères organiques et sa surface présente des fonctions chimiques appropriées.

4. Procédé selon la revendication 2 ou 3, caractérisé en ce que la fonctionnalisation des surfaces s'effectue par introduction de groupes aminopropyle, de groupes d'ancrage glycine ou de groupes d'ancrage diglycine.

5. Procédé selon la revendication 3 ou 4, caractérisé en ce que (a) on relie les peptides à synthétiser par l'extrémité C terminale directement avec le matériau plat ou (b) on établit une liaison avec le matériau plat par l'intermédiaire d'un lieur.

6. Procédé selon l'une des revendications précédentes, caractérisé en ce que la charge spécifique de surface ayant des fonctions réactives est de 1 pmol à 1 µmol par cm².

7. Procédé pour la synthèse rapide d'oligonucléotides libres ou immobilisés sur des substrats, dans lequel on part d'un ou plusieurs support(s) fonctionnalisé(s) et, par étapes, en fonction des séquences nucléotidiques prédéfinies, on synthétise les oligonucléotides souhaités selon un processus de synthèse approprié, caractérisé en ce qu'on
- synthétise simultanément, en parallèle, d'une pluralité à une multitude d'oligonucléotides différents, sur des surfaces de réaction en forme de points ou de stries n'entrant pas en contact les unes avec les autres,
- utilise un matériau plat comme support,
- applique à chaque étape de synthèse, pour le couplage, de petites quantités aliquotes des solutions des nucléotides souhaités ou des di- ou oligopeptides souhaités sous la forme de leurs dérivés activés, sous forme de points ou de stries sur les supports plats,
- effectue la réaction de couplage dans les surfaces réactionnelles qui se forment par imprégnation et
- sépare éventuellement du support plat les oligonucléotides formés au moyen d'une solution de séparation appropriée.

8. Procédé selon la revendication 7, caractérisé en ce qu'on utilise comme matériau plat une feuille ou une membrane poreuse ou non poreuse ou un papier, présentant des fonctions ayant une réactivité chimique, appropriées pour le couplage des nucléotides.

9. Procédé selon la revendication 7 ou 8, caractérisé en ce qu'on met en oeuvre les mesures de la revendication 3, 4 ou 6.

10. Procédé selon la revendication 9, caractérisé en ce que (a) on relie les oligonucléotides à synthétiser par l'extrémité 3' terminale directement avec le matériau plat ou (b) on effectue la liaison des oligonucléotides à synthétiser avec le matériau plat par l'intermédiaire d'un lieur.

11. Matériau plat sur lequel sont fixés des peptides, préparé selon l'une des revendications 1 à 6.

12. Matériau plat sur lequel sont fixés des oligonucléotides, préparé selon l'une des revendications 7 à 10.

13. Utilisation du matériau plat selon la revendication 11 à des fins microbiologiques et en particulier diagnostiques, en médecine humaine et vétérinaire, pour la détection spécifique d'infections bactériennes et virales.

14. Utilisation du matériau plat selon la revendication 12 à des fins d'hybridation.
